# EUROPEAN PATENT APPLICATION

(11) **EP 4 640 157 A1**
(43) Date of publication of application: **29.10.2025**
(21) Application number: 25171978.7
(22) Date of filing: 23.04.2025
(51) Int. Cl.: A61B 6/10, A61B 6/46, A61B 6/00, A61B 6/58

(54) **RADIOGRAPHIC IMAGING APPARATUS OF MOBILE TYPE, PROGRAM, AND MOVING IMAGE CAPTURING METHOD**

(30) Priority: 25.04.2024 JP 2024071577; 25.04.2024 JP 2024071584
(71) Applicant: KONICA MINOLTA, INC., Tokyo 100-7015 (JP)
(72) Inventor: YAMAMURA, Takuya, Tokyo, 100-7015 (JP); ONO, Kojiro, Tokyo, 100-7015 (JP)
(74) Representative: MFG Patentanwälte Meyer-Wildhagen Meggle-Freund Gerhard PartG mbB

(57) **Abstract**

Provided is a radiographic imaging apparatus of a mobile type that includes: an inputter that receives an input of a setting of moving image capturing; a controller that estimates a scattered radiation dose of the radiographic imaging apparatus of the mobile type based on the setting; and an outputter that outputs a scattered radiation dose map based on the scattered radiation dose.

## Description

### BACKGROUND OF THE INVENTION

### 1. Field of the Invention

The present disclosure relates to a radiographic imaging apparatus of a mobile type, a program, and a moving image capturing method.

### 2. Description of Related Art

As a radiographic imaging apparatus using radiation such as X-rays, a radiographic imaging apparatus of a mobile type is commercially available. The radiographic imaging apparatus of a mobile type is called a medical cart and can be used even in a general hospital room such as an ICU.

Imaging in a general hospital room is different from imaging in a radiation management area in that there is a possibility that a person such as a medical worker such as a nurse or a patient's family are present in the surroundings. For this reason, it is necessary to consider the exposure of persons present in the surroundings. An X-ray diagnostic apparatus is disclosed which displays a radiation dose map of a subject model and a scattered radiation dose map in the vicinity of the subject model, which are formed according to X-ray generation conditions, to enable easy management of exposure of the operator. As for the cumulative exposure dose, the movement trajectory of the subject is detected, and the position of the operator is stored in association with X-ray irradiation (for example, Japanese Patent Publication Laid-Open No. 2014-236798).

Although Japanese Patent Publication Laid-Open No. 2014-236798 makes it possible to perform exposure management of the operator by displaying the cumulative exposure dose to which the operator is exposed in the past imaging, it is not possible to estimate, before imaging, how much exposure will be due to the current imaging. In addition, although Japanese Patent Publication Laid-Open No. 2014-236798 makes it possible to manage an exposure dose in simple X-ray imaging, clinical use of X-ray dynamic imaging has been spreading in recent years.

### SUMMARY OF THE INVENTION

In radiographic dynamic imaging using radiation such as X-rays, a dynamic image constituted by a plurality of still images corresponding to each pulse is generated by a radiation generating apparatus repeatedly emitting radiation pulses at a period of a plurality of times per unit time (for example, 15 times per second) for a predetermined time (duration) while an emission instruction is given, and by a radiation detection apparatus reading out the amount of electric charge generated according to the dose of radiation received through the subject as a signal value (intensity). In moving image capturing such as fluoroscopic imaging or dynamic imaging, pulsed X-rays are emitted a plurality of times, and thus, it is desirable to estimate an exposure dose before imaging. That is, in dynamic imaging, since the exposure dose changes according to imaging conditions such as the imaging time, it is required to estimate the integrated radiation dose according to the imaging conditions.

Further, in imaging by a medical cart outside the radiation management area, there are various external factors such as the layout of the room of the imaging location and the arrangement of the patient bed, and the situation of scattering also varies depending on the imaging location, and thus, it is necessary to present an appropriate evacuation place to the operator and surrounding persons.

A radiographic imaging apparatus of a mobile type according to an aspect of the present disclosure includes: an inputter that receives an input of a setting of moving image capturing; a controller that estimates a scattered radiation dose of the radiographic imaging apparatus of the mobile type based on the setting; and an outputter that outputs a scattered radiation dose map based on the scattered radiation dose.

A program according to an aspect of the present disclosure causes a computer to execute: inputting a setting of moving image capturing; estimating a scattered radiation dose of a radiographic imaging apparatus of a mobile type based on the setting; and outputting a scattered radiation dose map based on the scattered radiation dose.

A moving image capturing method according to an aspect of the present disclosure includes: inputting a setting of moving image capturing; estimating a scattered radiation dose of a radiographic imaging apparatus of a mobile type based on the setting; outputting a scattered radiation dose map based on the scattered radiation dose; and capturing a moving image.

Note that, these generic or specific aspects may be implemented as a system, an apparatus, a method, an integrated circuit, a computer program, or a recording medium, or any selective combination of a system, an apparatus, a method, an integrated circuit, a computer program, and a recording medium.

### Advantageous Effects of Invention

According to an exemplary embodiment of the present disclosure, it is possible to present an appropriate evacuation place to the operator and surrounding persons by presenting a scattered radiation dose corresponding to an external situation when imaging is performed by a medical cart outside a radiation management area.

### BRIEF DESCRIPTION OF DRAWINGS

The advantages and features provided by one or more embodiments of the invention will become more fully understood from the detailed description given hereinbelow and the appended drawings which are given by way of illustration only, and thus are not intended as a definition of the limits of the present invention:
Fig. 1 is a diagram illustrating an example of a medical cart in use;
Fig. 2 is a diagram illustrating an example of the medical cart while moving or not in use;
Fig. 3 is a diagram illustrating functional blocks of the medical cart;
Fig. 4 is a diagram in which an estimated result is displayed;
Fig. 5 is a diagram in which directions with a small scattered radiation dose are highlighted and illustrated;
Fig. 6 is a diagram illustrating a display example of a scattered radiation dose according to an imaging time; and
Fig. 7 is a diagram illustrating a flowchart of processing performed by a controller.

### DETAILED DESCRIPTION OF EMBODIMENTS

Hereinafter, one or more embodiments of the present invention will be described with reference to the drawings. However, the scope of the invention is not limited to the disclosed embodiments.

Hereinafter, an embodiment of the present disclosure will be described in detail with reference to the accompanying drawings as appropriate.

### <1. Radiographic Imaging System>

First, a schematic configuration of a radiographic imaging apparatus according to an embodiment of the present disclosure will be described. The radiographic imaging apparatus includes at least a radiation generating apparatus. The radiographic imaging apparatus may be, for example, a medical cart 100. The medical cart 100 may have an autonomous driving function. The radiographic imaging apparatus may be a fixed radiographic imaging apparatus.

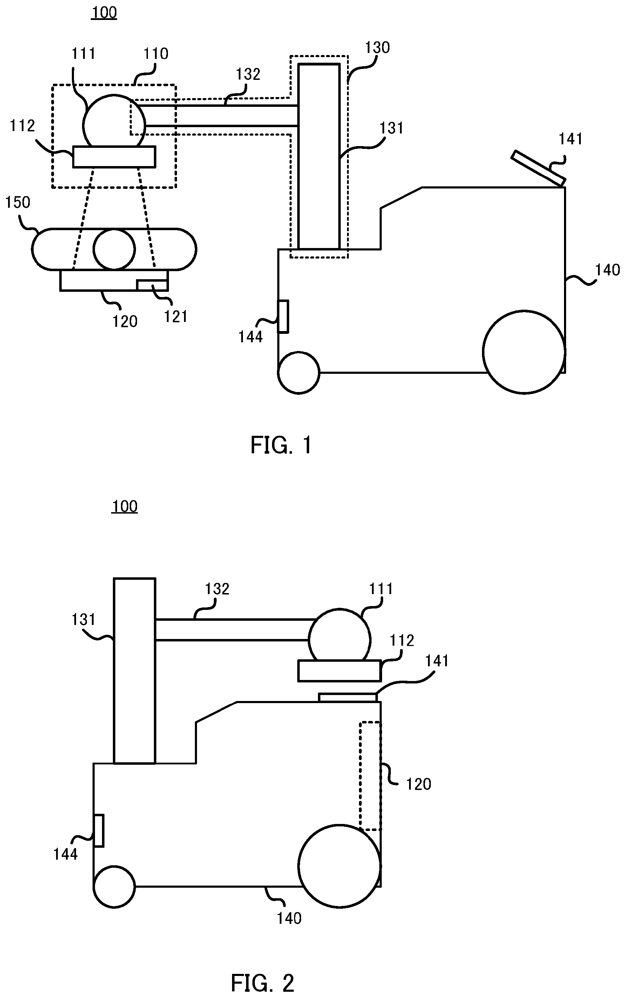

Fig. 1 is a diagram illustrating an example of the medical cart 100 in use.

Fig. 2 is a diagram illustrating an example of medical cart 100 while moving or not in use.

The medical cart 100 includes a radiation generating apparatus 110, a radiation detection apparatus 120, an arm 130, and a main body 140.

The medical cart 100 may be communicable with a hospital information system (HIS; not illustrated), a radiology information system (RIS; not illustrated), a picture archiving and communication system (PACS; not illustrated), other radiographic imaging apparatuses (not illustrated), and/or the like.

The medical cart 100 can be set to a plurality of imaging modes including a dynamic imaging mode for performing dynamic imaging and a fluoroscopic imaging mode for performing fluoroscopic imaging.

### [Radiation Generating Apparatus]

The radiation generating apparatus 110 includes a generator 111 and a collimator 112. The radiation generating apparatus 110 may be a radiation generating unit.

The generator 111 includes a tube. When a voltage is applied to the tube and a current flows through the tube, radiation, for example, X-rays, is emitted from the tube. A pulsed voltage may be applied to the tube, and pulsed radiation, for example, X-rays, may be emitted from the tube.

The maximum radiation dose to be emitted, that is, the allowable radiation dose of the generator 111, is limited according to the imaging mode of moving image capturing (for example, the dynamic imaging mode or the fluoroscopy mode) set to the medical cart 100. In the dynamic imaging mode, the radiation dose to be emitted is limited due to limitations as a general imaging apparatus. In the fluoroscopy mode, there are no limitations as a general imaging apparatus, and thus, a larger radiation dose can be emitted than in the dynamic imaging mode. In the fluoroscopy mode, the radiation dose may not be limited. Further, the generator 111 emits radiation based on the setting through the inputter. The imaging conditions are, for example, the conditions related to a subject 150, such as the imaging site, the imaging direction, and the physique, and the conditions related to emission of radiation, such as the tube voltage, the tube current, the emission time, the current-time product (mAs value), the frame rate, the number of allowable frames, the pulse width, the pulse interval, the pulse period, the pulse duty ratio, the focus to object distance (FOD), and the irradiation field range (the degree of aperture of the collimator 112).

In dynamic imaging, that is, in imaging in the dynamic imaging mode, the radiation generating apparatus 110 repeatedly emits radiation pulses at a period of a plurality of times per unit time (for example, 15 times per second) for a predetermined time (duration) while an emission instruction is given. Here, the pulse period and the duration are set in advance by an inputter/outputter 141. The pulse interval, the pulse width, and the pulse duty ratio may be set in advance by the inputter/outputter 141. The radiation detection apparatus 120 acquires an image corresponding to each pulse to acquire a series of a plurality of images (dynamic images). Note that, in the dynamic imaging mode in the present embodiment, a configuration in which radiation pulses are emitted will be described as an example, but a configuration in which radiation is continuously emitted may also be employed. In the dynamic imaging mode, emission of radiation is limited due to limitations as a general imaging apparatus.

In the fluoroscopy, that is, in imaging in the fluoroscopy mode, the radiation generating apparatus 110 repeatedly emits radiation pulses at a period of a plurality of times per unit time (for example, 6 times, 10 times, or 15 times per second) while an emission instruction is given. Here, the pulse period is set in advance by the inputter/outputter 141. In the fluoroscopy mode, no duration is set. That is, in the fluoroscopy mode, emission of radiation is not limited by the duration. Further, in the fluoroscopy mode, there are no limitations as a general imaging apparatus, and thus, more radiation can be emitted than in the dynamic imaging mode. In the fluoroscopy mode, the radiation dose may not be limited. The pulse interval, the pulse width, and the pulse duty ratio may be set in advance by the inputter/outputter 141. The radiation detection apparatus 120 detects an image corresponding to each pulse, and the inputter/outputter 141 displays the image. Note that, in the fluoroscopy mode in the present embodiment, a configuration in which radiation pulses are emitted will be described as an example, but a configuration in which radiation is continuously emitted may also be employed.

The collimator 112 narrows an irradiation field of emitted radiation. The collimator 112 may include a shielding means such as a filter. The shielding means may narrow a region to be irradiated with emitted radiation. The shielding means may weaken the intensity of emitted radiation.

### [Radiation Detection Apparatus]

The radiation detection apparatus 120 includes a communicator 121. Further, although not illustrated, the radiation detection apparatus further includes a sensor board, a scanning circuit, a readout circuit, a controller, and the like. The radiation detection apparatus 120 may be a radiation detection unit.

The radiation detection apparatus 120 detects radiation emitted from the radiation generating apparatus 110 via the subject 150. The subject 150 is, for example, a human, an animal, or the like.

In the sensor board, pixels are arranged two-dimensionally (in a matrix). Each pixel includes a radiation detection element, which generates electric charge corresponding to the dose of radiation received via a subject, and a switch element, which accumulates and releases the electric charge. The scanning circuit turns on or off each switch element. The readout circuit reads out the amount of electric charge released from each pixel as a signal value (intensity). The controller controls the radiation detection apparatus 120 in its entirety. The controller causes a radiographic image to be generated from a plurality of signal values read out by the readout circuit. The communicator 121 transmits the data of the radiographic image generated by the readout circuit, various signals, and the like to the outside. Further, the communicator 121 receives various pieces of information and various signals. The communicator 121 may perform wireless communication or may perform wired communication.

In each pixel of the radiation detection apparatus 120 configured in such a manner, when the radiation detection element receives radiation in a state in which the scanning circuit turns off the switch element, the radiation detection element generates electric charge corresponding to the dose of radiation and accumulates the electric charge. When the scanning circuit turns on the switch element, the accumulated electric charge is released, and the readout circuit detects the amount of electric charge released from each pixel and generates a signal value indicating the amount of electric charge. The controller generates a radiographic image based on the signal value generated for each pixel. The generated individual radiographic images are one still image in the dynamic imaging. In the case of pulsed radiation, one still image is generated correspondingly to each pulse.

The communicator 121 communicates with a communicator 144 of the main body 140 and transmits a generated radiographic image to the communicator 144 of the main body 140. The communicator 121 may transmit a still image to the main body 140 each time one still image is generated, or may collectively transmit a plurality of still images to the communicator 144 of the main body 140. The communicator 121 may communicate with a component other than the main body 140. The communication performed by the communicator 121 may be wireless communication or wired communication.

In a case where the radiation generating apparatus 110 performs pulse emission, the timing at which the radiation detection apparatus 120 generates a plurality of still images constituting a dynamic image is synchronized with the timing at which radiation is emitted from the radiation generating apparatus 110. In a case where the radiation generating apparatus 110 performs continuous emission, on the other hand, the timing at which the radiation detection apparatus 120 generates a plurality of still images constituting a dynamic image is arbitrary timing during the time of the continuous emission.

The radiation detection apparatus 120 may be stored in a storage provided in the main body 140 when the medical cart 100 is moved or not in use. Fig. 2 illustrates how the radiation detection apparatus 120 is stored in the storage of the main body 140 with a dotted line. When the radiation detection apparatus 120 is stored in the storage of the main body 140, the radiation detection apparatus 120 may be connected to the main body 140 in a wired manner, and the radiation detection apparatus 120 may be charged from the main body 140 and may communicate with the main body 140. When the radiation detection apparatus 120 is stored in the storage of the main body 140, software or firmware of the radiation detection apparatus 120 may be updated by communication with the main body 140. The radiation detection apparatus 120 may be charged and communicate with the main body 140 wirelessly.

The radiation detection apparatus 120 may be wirelessly connected when in use. The radiation detection apparatus 120 may be driven by an internal battery. Power may be supplied to the radiation detection apparatus 120 from the main body 140 in a wired manner or wirelessly.

The radiation detection apparatus 120 may be connected to the main body 140 in a wired manner when moving or not in use, and may be connected to the main body 140 wirelessly when in use.

### [Arm]

The arm 130 includes a vertical arm 131 and a horizontal arm 132. The vertical arm 131 pivotably supports the horizontal arm 132, that is, in a rotatable manner with respect to the Z-axis. Further, the vertical arm 131 may movably support the horizontal arm 132. The horizontal arm 132 pivotably supports the radiation generating apparatus 110, that is, in a rotatable manner with respect to the X-axis and the Y-axis. The radiation generating apparatus 110 can be oriented in any direction with respect to the main body 140 by the vertical arm 131 and the horizontal arm 132. The position and orientation of the arm may be determined by an input through the inputter/outputter 141. The vertical arm 131 and the horizontal arm 132 may be rotatable and movable by the control by the main body 140 or may be rotatable and movable manually.

### [Main Body]

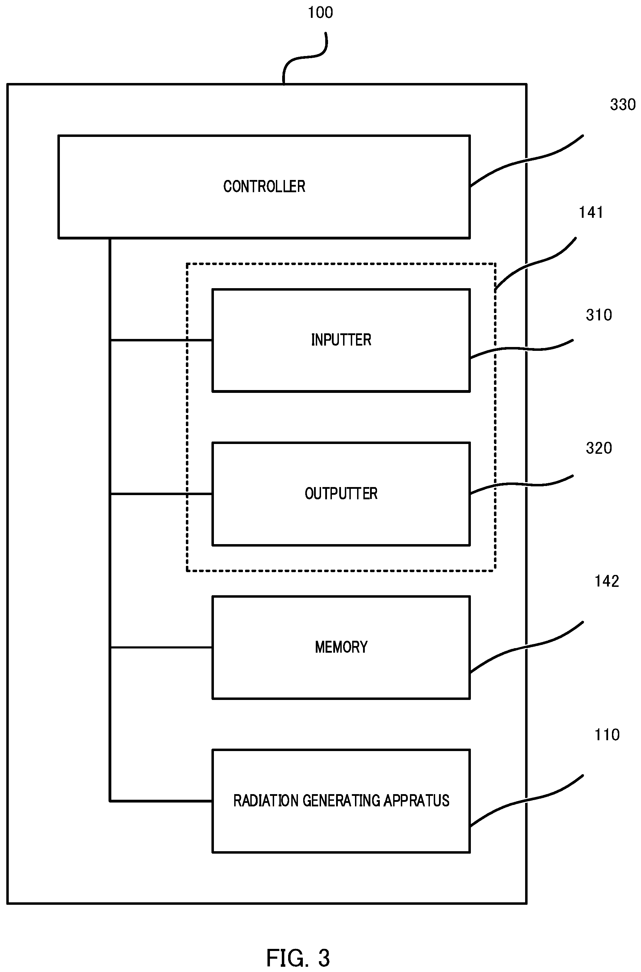

The main body 140 includes the inputter/outputter 141 and the communicator 144. The main body 140 may include a controller (control circuit) 330 and a memory 142 which are illustrated in Fig. 3. Although not illustrated, the main body 140 may include a storage that stores the radiation detection apparatus 120.

The inputter/outputter 141 may have a variable inclination angle with respect to the main body 140. The inputter/outputter 141 may be separable from the main body 140. In a case where the inputter/outputter 141 is separated from the main body 140, the inputter/outputter 141 and the main body 140 are connected to each other wirelessly or in a wired manner.

The inputter/outputter 141 may be constituted by a touch screen, a keyboard, a mouse, a microphone, a camera, a display, a speaker, a display lamp, and/or the like. The inputter/outputter 141 may be divided into an inputter and an outputter. For the inputter/outputter 141, an input means for an input by sound, an input by gesture, an input by line of sight, and an input by brain waves and/or the like may be available. The inputter/outputter 141 performs settings of the radiation generating apparatus 110. Further, the inputter/outputter 141 makes it possible to perform an operation of instructing the radiation generating apparatus 110 to emit radiation. The inputter/outputter 141 may output an emission instruction during a period from when a toggle button is pressed for the first time to when the toggle button is pressed for the second time, or may output an emission instruction during a period in which the button is continuously pressed.

When dynamic imaging is performed, the inputter/outputter 141 receives an input of an imaging condition(s). The imaging conditions include the imaging time, the frame rate, the tube voltage, the tube current, or the tube voltage-time product (mAs). The inputted imaging condition(s) is/are set to the radiation generating apparatus 110. The imaging condition(s) may be determined according to the imaging order. The imaging condition(s) determined according to the imaging order may be changeable (modifiable).

When dynamic imaging is performed, the inputter/outputter 141 receives an input of an external factor(s). The external factors include the layout of the imaging location, the arrangement of the patient bed, the position of the shielding plate, or the material of the shielding plate. The external factor(s) may be determined according to the imaging order. The external factor(s) determined according to the imaging order may be modifiable. The external factor(s) may be generated by analyzing an image captured by a camera mounted in the medical cart 100 or by analyzing information acquired by a sensor mounted in the medical cart 100. The external factor(s) generated based on the captured image or the information acquired by the sensor may be modifiable.

In response to an operation of the inputter/outputter 141, the generator 111 emits radiation of a dose set by the inputter/outputter 141. The radiation is, for example, X-rays.

The inputter/outputter 141 may issue a warning. Note that, the notification will be described in detail later.

The communicator 144 communicates with the radiation detection apparatus 120. The communication between the communicator 144 of the main body 140 and the communicator 121 of the radiation detection apparatus 120 may be wireless communication or wired communication.

### [Functional Blocks]

Fig. 3 is a diagram illustrating functional blocks of the medical cart 100. The medical cart 100 includes an inputter 310, an outputter 320, the memory 142, the radiation generating apparatus 110, and the controller 330. The inputter 310 and the outputter 320 constitutes the inputter/outputter 141. The inputter 310 and the outputter 320 may be integrated.

When the imaging start is instructed, the inputter 310 may notify the controller 330 that the imaging start has been instructed.

The outputter 320 displays a notification regarding a scattered radiation dose by the controller 330.

The memory 142 stores programs, parameters, and the like used by the controller 330 for processing. The memory 142 may store maps in which the layout of the imaging location, the arrangement of the patient bed, and the like (hereinafter each map will be referred to as a base map) are stored. The memory 142 may store two-dimensional base maps and three-dimensional base maps. The memory 142 may store base maps corresponding to cases where imaging is performed in the sitting position and base maps corresponding to cases where imaging is performed in the lying position. The memory 142 may store base maps in which nothing is set.

The radiation generating apparatus 110 emits radiation based on the imaging conditions such as the imaging time, the frame rate, the tube voltage, the tube current, and the tube voltage-time product (mAs).

The controller 330 controls the medical cart 100 in its entirety. The controller 330 is constituted by a computer, such as a central processing unit (CPU), an application specific integrated circuit (ASIC), or a field programmable gate array (FPGA).

The controller 330 may be instructed to start imaging by the inputter 310. The controller 330 may be present in the main body 140. The controller 330 may select and acquire, from the memory 142, a base map corresponding to the location where the medical cart 100 is present. According to whether the imaging is performed in the sitting position or in the lying position, the controller 330 may select and acquire, from the memory 142, a base map corresponding to a case where imaging is performed in the sitting position or a base map corresponding to a case where imaging is performed in the lying position. The controller 330 may select and acquire a two-dimensional base map or a three-dimensional base map according to an input through inputter 310.

The controller 330 estimates the scattered radiation dose based on the imaging conditions inputted through the inputter/outputter 141 and causes the outputter 320 to display the estimation result before imaging. The controller 330 calculates the dose of radiation to be emitted, based on the tube voltage, the tube current, the emission time, the current-time product (mAs value), the frame rate, the number of allowable frames, the pulse width, the pulse interval, the pulse period, the pulse duty ratio, and/or the like. The controller 330 calculates a region where radiation is emitted, based on the imaging site, the imaging direction, the FOD, the irradiation field range, and/or the like. The controllers 330 can estimate the scattered radiation dose scattered in a region other than the region where radiation is emitted, based on the dose of radiation to be emitted and the region where radiation is emitted. The controller 330 can estimate the amounts of the emitted radiation to be reflected and absorbed, based on the layout of the imaging location, the conditions related to the subject 150 such as the physique, the arrangement of the patient bed, the position of the shielding plate, the material of the shielding plate, and/or the like. The controller 330 may estimate the scattered radiation dose scattered in a region other than the region where radiation is emitted, in consideration of the amounts of radiation to be reflected and absorbed in addition to the dose of radiation to be emitted and the region where radiation is emitted. In response to an instruction to start imaging inputted through the inputter 310, the controller 330 may cause the estimation result to be displayed, and may cause the radiation generating apparatus 110 to emit radiation in a case where it is confirmed that the operator and surrounding persons have been evacuated. The controller 330 may cause an estimated result to be displayed in a case where a setting is inputted through the inputter 310, and may enable an instruction to start imaging to be given in a case where it is confirmed that the operator and surrounding persons have been evacuated.

The controller 330 may perform the estimation in consideration of the subject situations such as the imaging body position (standing position, sitting position, lying position, or the like) of the subject 150, the imaging location of the subject 150, the body thickness of the subject 150, the material of the bed, the orientation of the arm, the position of the bed, the angle of the bed, and the position of a monitor apparatus of the subject 150.

In imaging by a medical cart outside a radiation management area, there are various external factors such as the layout of the imaging location, the arrangement of the patient bed, and the shielding plate, and thus, it is necessary to present an appropriate evacuation place to the operator or surrounding persons.

### [Display]

### <Display Example 1>

The controller 330 determines the location where the medical cart 100 is present. The controller 330 may determine the location where the medical cart 100 is present, by using a global navigation satellite system (GNSS), such as a global positioning system (GPS), mounted in the medical cart 100, or may determine the location where the medical cart 100 is present, based on information acquired from a camera mounted in the medical cart 100. The controller 330 may determine the location where the medical cart 100 is present by the medical cart 100 receiving a beacon disposed in the hospital.

The controller 330 may select a base map according to the determined imaging location of the medical cart 100. The controller 330 may create a base map based on information acquired from a camera and a sensor mounted in the medical cart 100. For example, the controller 330 may select a base map according to the body position of the subject 150. The controller 330 may select a base map corresponding to the imaging location or the like and modify the selected base map according to the imaging condition(s), external factor(s), and/or the like.

The controller 330 estimates the scattered radiation dose by performing simulation based on the imaging conditions such as the imaging time, the frame rate, the tube voltage, the tube current, and the tube voltage-time product (mAs), which have been set to the radiation generating apparatus 110. The controller 330 may estimate the scattered radiation dose in consideration of the external factors such as the layout of the imaging location, the material of the bed, the imaging site (chest/abdomen, four limbs, or the like), the irradiation direction of radiation, the body thickness, the position (orientation) of the arm, the positions (layouts) of the medical cart and the bed, and the positions of other monitors such as a respiration monitor.

The controller 330 estimates the scattered radiation dose of radiation emitted by the radiation generating apparatus 110, and overlaps the estimation result on the base map. In the scattered radiation dose map that the controller 330 causes the inputter/outputter 141 to display, the estimated scattered radiation dose is overlapped on a base map. The controller 330 may cause the estimated result to be displayed two-dimensionally or three-dimensionally. The controller 330 may cause a region (direction) with a small scattered radiation dose and/or the evacuation place to be highlighted and displayed on the screen, to be outputted by sound, or to be outputted by both displaying on the screen and sound. For example, the controller 330 may cause the orientation of the map to be changed and causes the map to be displayed such that a region (direction) with a small scattered radiation dose and/or the evacuation place is/are displayed in an upper portion of the screen. For example, the controller 330 may cause a region with a small scattered radiation dose to be displayed on the screen by changing the color of the region or surrounding the region with a line. For example, the controller 330 may cause a region with a small scattered radiation dose to be displayed in another window. Another window may be disposed, for example, in an upper portion of the screen. The controller 330 may cause a scattered radiation dose to be displayed with a color(s) corresponding to the scattered radiation dose or may cause only a scattered radiation dose exceeding a predetermined radiation dose or only a scattered radiation dose equal to or less than the predetermined radiation dose to be displayed. The controller 330 may cause a scattered radiation dose to be displayed with a color(s) corresponding to the scattered radiation dose or may cause a change between the display aspect of a scattered radiation dose exceeding a predetermined radiation dose and the display aspect of a scattered radiation dose equal to or less than a predetermined radiation dose to be performed. The correspondence between the scattered radiation dose and the color(s) may be changeable. The controller 330 may change the predetermined radiation dose according to the determined location where the medical cart 100 is present. The controller 330 may change the map according to the movement of the arm 130. The controller 330 may change the orientation of a scattered radiation dose map to be displayed (the direction of an upper portion of the map) according to the orientation of the medical cart 100, the orientation of the arm 130, the irradiation direction of radiation, or the orientation of the subject 150. The controller 330 may estimate the scattered radiation dose in consideration of the disposed shielding material. The controller 330 may estimate the scattered radiation dose in conjunction with the movement of the arm 130 of the medical cart 100.

In a case where an estimated scattered radiation dose is greater than a value set in advance, the controller 330 may cause the outputter 320 to output a warning. The warning can be performed by sound and/or displaying on the screen. In a case where the controller 330 recognizes, by using a camera mounted in the medical cart 100, that a person is present at a position where the scattered radiation dose is greater than a set value, the controller 330 may cause a warning to be outputted. The controller 330 may cause a warning to be outputted which prompts the user to change the imaging conditions.

As the warning, for example, "Imaging is feasible", "The scattered radiation dose is too much", "Please confirm evacuation", "Please install the shielding plate", "Please change the imaging conditions", or the like may be outputted. As the warning, a warning at an elapsed time point may be displayed, a warning for the entire imaging may be displayed, or both the warnings may be displayed. The controller 330 may select a warning in consideration of the imaging conditions for obtaining the required image quality. For example, in a case where it is not possible to lower the imaging conditions in order to obtain the required image quality, "Please install the shielding sheet" may be selected instead of "Please change the imaging conditions".

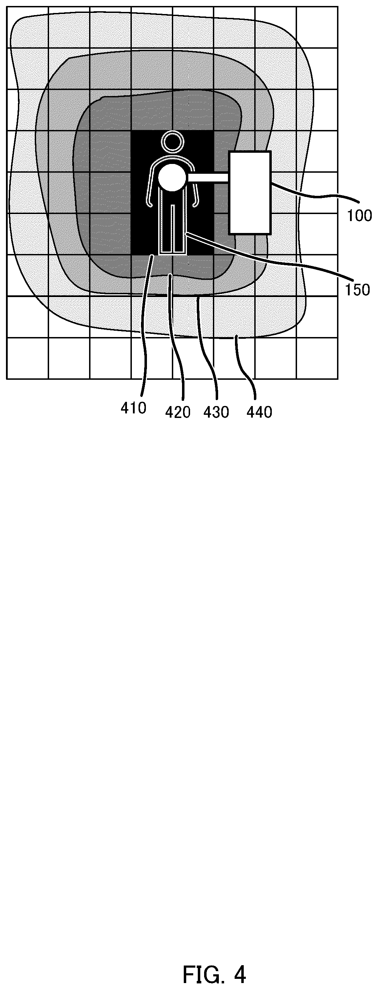

Fig. 4 is a diagram in which an estimated result is illustrated. Fig. 4 illustrates the scattered radiation dose around a bed 410 on which the subject 150 is lying. The subject 150 is lying on the bed 410 with the head on the upper side and the legs on the lower side. Further, the medical cart 100 is present on the right side of the bed 410. Note that, the medical cart 100 may be present on the left side of the bed 410. The result of the estimated scattered radiation dose may be displayed with contour lines using micro Sievert. For example, a line 420 represents eight micro Sievert, a line 430 represents two micro Sievert, and a line 440 represents one micro Sievert. For example, a region with a scattered radiation dose higher than the line 420 may be displayed in red, a region with a scattered radiation dose higher than the line 430 may be displayed in orange, and a region with a scattered radiation dose higher than the line 440 may be displayed in yellow. Although Fig. 4 illustrates a state in which the scattered radiation dose is displayed two-dimensionally, the scattered radiation dose may also be displayed three-dimensionally. In Fig. 4, for example, one cell is one meter. The number of regions to be displayed is arbitrary, and the number of contour lines using micro Sievert of scattered radiation to be displayed as well as the specific scattered radiation dose to be displayed are also arbitrary. The scattered radiation dose may be displayed by a color gradation corresponding to the scattered radiation dose. Further, Fig. 4 illustrates an estimation result when the imaging time is 15 seconds.

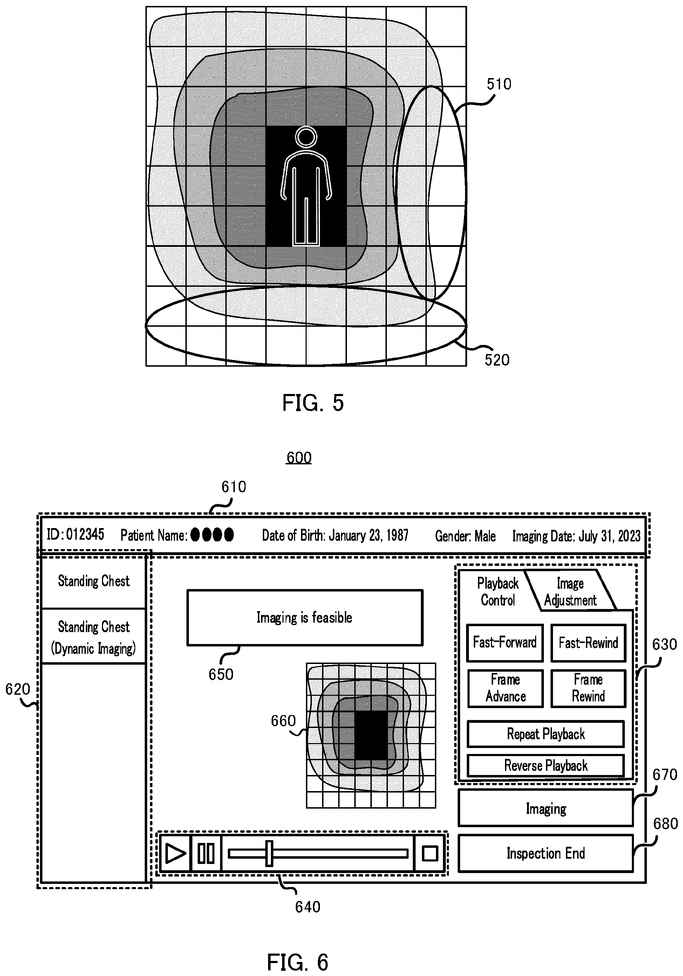

Fig. 5 is a diagram in which regions (directions) with a small scattered radiation dose are highlighted and illustrated. Regions 510 and 520 with a small scattered radiation dose are overlapped, based on the estimation result in Fig. 4, with the estimation result (Fig. 4). For example, the frames of the regions 510 and 520 may be displayed in blue. In Fig. 5, a region with two micro Sievert or less is configured as a region with a small scattered radiation dose, but the region with a small scattered radiation dose may be a region with one micro Sievert or less or the small scattered radiation value may be another value. Further, sound such as "Please move one meter or more away from the bed to the right of the bed or to this side of the bed" or the like may be outputted.

### <Display Example 2>

In the display example 2, displaying is performed in consideration of the imaging time for a moving image. In addition to the displaying described in the display example 1, displaying may be performed in consideration of the imaging time.

In a case where dynamic imaging is performed, the radiation dose to which the operator such as an X-ray technician or a doctor is exposed increases with the imaging time. Accordingly, it is necessary to estimate the scattered radiation dose according to the imaging time and provide an evacuation place.

Fig. 6 is a diagram illustrating a display example of a scattered radiation dose according to an imaging time.

The display 600 may be displayed on the touch screen of the inputter/outputter 141. When a content displayed on the display 600 is selected, the control part 330 performs corresponding control.

The display 600 includes bibliographic information 610, imaging conditions 620, playback control 630, a playback state 640, a warning 650, a scattered radiation dose 660, imaging 670, and an inspection end 680. The display 600 may include other information such as "change in imaging conditions" and "evacuation is completed", or may not include some information.

The bibliographic information 610 may include the patient ID, the patient name, the date of birth of the patient, the gender of the patient, and the imaging date. The bibliographic information 610 may include other information such as the department in charge and the name of the doctor in charge.

The imaging conditions 620 may include the imaging site (chest, stomach, or the like), the imaging body position (standing position, sitting position, lying position, or the like), and the imaging type (fluoroscopy, dynamic imaging, still image, or the like). The imaging conditions 620 may include the imaging time, the frame rate, the tube voltage, the tube current, the tube voltage-time product (mAs), the FOD, the irradiation field range, and the like.

The playback control 630 may include playback types such as fast-forward, fast-rewind (rewind), frame advance, frame rewind, repeat playback, and reverse playback. The playback control 630 may allow image adjustment to be performed using tabs. The image adjustment may include a change in imaging conditions. Instead of selecting playback types and image adjustment by the tabs, each may be displayed separately. For example, when the "fast-forward" of the playback control 630 is selected, the elapsed time advances at, for example, double speed, and the scattered radiation dose that has changed with the advance of time is displayed. For example, when the "frame advance" of the playback control is selected, the scattered radiation dose in the elapsed time for each fixed time is displayed.

The playback state 640 may include the playback, the pause, the current state, and the end. In the current state, a horizontal bar may indicate the total imaging time, and the position of a vertical bar may indicate the elapsed time with respect to the total imaging time. The controller 330 may advance the elapsed time when the playback is selected, may stop the advance of the elapsed time when the pause is selected, and may end the display of the scattered radiation dose when the end is selected. For example, when the "playback" of the playback state 640 is selected, the elapsed time advances, the position of the vertical bar of the current state moves to the right, and the scattered radiation dose 660 that has changed with the advance of time is displayed.

The warning 650 includes a warning. As the warning, for example, "Imaging is feasible", "The scattered radiation dose is too much", "Please confirm evacuation", "Please install the shielding plate", "Please change the imaging conditions", or the like may be displayed. As the warning 650, a warning at an elapsed time point may be displayed, a warning for the entire imaging may be displayed, or both the warnings may be displayed.

The scattered radiation dose 660 indicates a scattered radiation dose map at the elapsed time indicated by the playback state 640. The scattered radiation dose 660 may indicate a scattered radiation dose map according to the imaging time. A scattered radiation dose map corresponding to a plurality of imaging times may be displayed. Scattered radiation dose maps with different imaging times may be selectable. In a case where a setting change has been performed, the scattered radiation dose (history) before the change may be displayed together with the current scattered radiation. By displaying a scattered radiation dose map corresponding to a plurality of imaging conditions, it is possible to perform imaging in consideration of exposure of surrounding persons. In a case where the controller 330 determines that the scattered radiation dose is smaller than a predetermined radiation dose, such as in a case where the imaging time is short or in a case where a still image is captured, the controller 330 may not cause the scattered radiation dose map to be displayed. In a case where the controller 330 determines that the scattered radiation dose is larger than a predetermined radiation dose, such as in a case where the imaging time is equal to or longer than a predetermined time, the controller 330 may cause the scattered radiation dose map to be displayed without fail and may cause imaging to be performed after the scattered radiation dose map is confirmed, that is, after safety of the operator and surrounding persons is confirmed.

When the imaging 670 is selected, imaging is performed. For example, when the operator touches a button for the imaging 670 displayed on the touch screen, the medical cart 100 starts imaging. The imaging 670 may be selectable after the scattered radiation dose 660 is confirmed, or may be selectable after one scattered radiation dose map is selected from a plurality of scattered radiation dose maps. The button for the imaging 670 may be selectable after it is confirmed that the evacuation is completed.

When the inspection end 680 is selected, the inspection ends. The power supply of the medical cart 100 may be turned off by selecting the inspection end 680.

### [Flowchart]

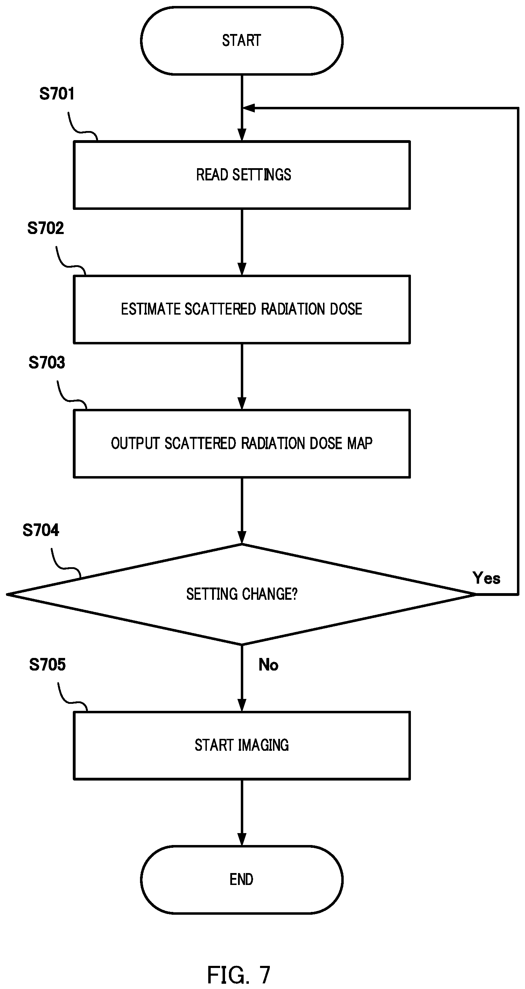

Fig. 7 is a diagram illustrating a flowchart of processing performed by the controller 330.

The controller 330 reads the settings (step S701). The setting includes imaging conditions, external factors, and subject situations. The settings may be read based on the imaging order. The correspondence between the imaging order and the settings may be stored in the memory 142. The imaging conditions may include, for example, conditions related to the subject 150, such as the imaging site, the imaging direction, and the physique, the tube voltage, the tube current, the emission time, the current-time product (mAs value), the frame rate, the number of allowable frames, the pulse width, the pulse interval, the pulse period, the pulse duty ratio, the FOD, the irradiation field range, and the like. The external factors may include the layout of the imaging location, the arrangement of the patient bed, the shielding plate, and the like. The subject situations may include the imaging body position (standing position, sitting position, lying position, or the like) of the subject 150, the imaging location of the subject 150, the body thickness of the subject 150, the material of the bed, the orientation of the arm, the position of the bed, the angle of the bed, the position of the monitor apparatus with respect to the subject 150, and the like.

The controller 330 estimates the scattered radiation dose based on the read settings (step S702).

The controller 330 causes the estimated scattered radiation dose to be displayed on a base map to create and output a scattered radiation dose map (step S703).

The controller 330 determines whether a setting change has been performed (step S704). In a case were a setting change has been performed (Yes in step S704), the processing returns to step S701, and the changed setting is read. In a case where a setting change has not performed (No in step S704), imaging is started (step S705). The controller 330 may confirm that an evacuation has taken place at the start of the imaging. The imaging includes fluoroscopic imaging or dynamic imaging.

In a case where a setting change has been performed, the imaging conditions of the radiation generating apparatus may be changed at the same time as the change, or the imaging conditions of the radiation generating apparatus may be changed when imaging is performed.

Upon completion of the imaging, the controller 330 ends the processing.

The processing described in the present disclosure is executed by a program.

In the present disclosure, the medical cart 100, which is a radiographic imaging apparatus of a mobile type, has been described. However, the scattered radiation dose can also be estimated and displayed in the same manner for a radiographic imaging apparatus of a fixed type. Further, although it has been described that the controller 330 estimates the scattered radiation dose and causes the scattered radiation dose to be displayed when dynamic imaging is performed, the scattered radiation dose can also be similarly estimated and displayed when fluoroscopy is performed.

Although an embodiment has been described above with reference to the accompanying drawings, the present disclosure is not limited to such an example. It is obvious that a person skilled in the art can conceive of various change examples or modification examples within the scope described in the claims. It is to be understood that such change examples or modification examples also belong to the technical scope of the present disclosure. Further, the constituent elements in the embodiment may be arbitrarily combined without departing from the spirit of the present disclosure.

According to the present disclosure, an appropriate evacuation place can be presented.
(1) A radiographic imaging apparatus of a mobile type according to an aspect of the present disclosure includes: an inputter that receives an input of a setting of moving image capturing; a controller that estimates a scattered radiation dose of the radiographic imaging apparatus of the mobile type based on the setting; and an outputter that outputs a scattered radiation dose map based on the scattered radiation dose.
(2) In the radiographic imaging apparatus of the mobile type according to an aspect of the present disclosure, in the radiographic imaging apparatus of (1), the controller estimates the scattered radiation dose in dynamic imaging by the radiographic imaging apparatus of the mobile type based on the setting.
(3) In the radiographic imaging apparatus of the mobile type according to an aspect of the present disclosure, in the radiographic imaging apparatus of (1), the scattered radiation dose map illustrates a region with a radiation dose equal to or less than a predetermined radiation dose.
(4) In the radiographic imaging apparatus of the mobile type according to an aspect of the present disclosure, in the radiographic imaging apparatus of (1), the scattered radiation dose map illustrates a region with a radiation dose exceeding a predetermined radiation dose.
(5) In the radiographic imaging apparatus of the mobile type according to an aspect of the present disclosure, in the radiographic imaging apparatus of (3) or (4), the predetermined radiation dose is changeable.
(6) In the radiographic imaging apparatus of the mobile type according to an aspect of the present disclosure, in the radiographic imaging apparatus of (1), the scattered radiation dose map is colored.
(7) In the radiographic imaging apparatus of the mobile type according to an aspect of the present disclosure, in the radiographic imaging apparatus of (1), the scattered radiation dose map is a scattered radiation dose map in which the scattered radiation dose is overlapped on a base map corresponding to the setting.
(8) In the radiographic imaging apparatus of the mobile type according to an aspect of the present disclosure, in the radiographic imaging apparatus of (1), the setting(s) includes an imaging location.
(9) In the radiographic imaging apparatus of the mobile type according to an aspect of the present disclosure, in the radiographic imaging apparatus of (8), the setting(s) includes an external factor(s) at the imaging location.
(10) In the radiographic imaging apparatus of the mobile type according to an aspect of the present disclosure, in the radiographic imaging apparatus of (9), the external factor(s) includes an arrangement of a shielding material.
(11) In the radiographic imaging apparatus of the mobile type according to an aspect of the present disclosure, in the radiographic imaging apparatus of (1), the setting(s) includes an imaging condition for a radiation generating apparatus.
(12) In the radiographic imaging apparatus of the mobile type according to an aspect of the present disclosure, in the radiographic imaging apparatus of (1), the setting(s) includes an imaging body position.
(13) A program according to an aspect of the present disclosure causes a computer to execute: inputting a setting of moving image capturing; estimating a scattered radiation dose of a radiographic imaging apparatus of a mobile type based on the setting; and outputting a scattered radiation dose map based on the scattered radiation dose.
(14) In the program according to an aspect of the present disclosure, in the program of (13), in the estimating, the scattered radiation dose in dynamic imaging by the radiographic imaging apparatus of the mobile type is estimated based on the setting.
(15) In the program according to an aspect of the present disclosure, in the program of (13), the scattered radiation dose map illustrates a region with a radiation dose equal to or less than a predetermined radiation dose.
(16) In the program according to an aspect of the present disclosure, in the program of (13), the scattered radiation dose map illustrates a region with a radiation dose exceeding a predetermined radiation dose.
(17) In the program according to an aspect of the present disclosure, in the program of (15) or (16), the predetermined radiation dose is changeable.
(18) In the program according to an aspect of the present disclosure, in the program of (13), the scattered radiation dose map is colored.
(19) In the program according to an aspect of the present disclosure, in the program of (13), the scattered radiation dose map is a scatted radiation dose map in which the scattered radiation dose is overlapped on a base map corresponding to the setting.
(20) In the program according to an aspect of the present disclosure, in the program of (13), the setting(s) includes an imaging location.
(21) In the program according to an aspect of the present disclosure, in the program of (20), the setting(s) includes an external factor(s) at the imaging location.
(22) In the program according to an aspect of the present disclosure, in the program of (21), the external factor(s) includes an arrangement of a shielding material.
(23) In the program acording to an aspect of the present disclosure, in the program of (13), the setting(s) includes an imaging condition for a radiation generating apparatus.
(24) In the program according to an aspect of the present disclosure, in the program of (13), the setting(s) includes an imaging body position.
(25) A moving image capturing method according to an aspect of the present disclosure includes: inputting a setting of moving image capturing; estimating a scattered radiation dose of a radiographic imaging apparatus of a mobile type based on the setting; outputting a scattered radiation dose map on which the scattered radiation dose is displayed; and capturing a moving image.

### Industrial Applicability

The present disclosure is useful for a medical cart.

Although embodiments of the present invention have been described and illustrated in detail, the disclosed embodiments are made for purpose of illustration and example only and not limitation. The scope of the present invention should be interpreted by terms of the appended claims.

### Reference Signs List

100 Medical cart
110 Radiation generating apparatus
111 Generator
112 Collimator
120 Radiation detection apparatus
121, 144 Communicator
130 Arm
131 Vertical arm
132 Horizontal arm
140 Main body
141 Inputter/outputter
142 Memory
150 Subject
310 Inputter
320 Outputter
330 Controller
410 Bed
420, 430, 440 Line
510, 520 Region
600 Display
610 Bibliographic information
620 Imaging conditions
630 Playback control
640 Playback state
650 Warning
660 Scattered radiation dose
670 Imaging
680 Inspection end

## Claims

1. A radiographic imaging apparatus of a mobile type (100), comprising:
an inputter (310) that receives an input of a setting of moving image capturing;
a controller (330) that estimates a scattered radiation dose (660) of the radiographic imaging apparatus of the mobile type based on the setting; and
an outputter (320) that outputs a scattered radiation dose map based on the scattered radiation dose (660).

2. The radiographic imaging apparatus of the mobile type according to claim 1, wherein
the controller (330) estimates the scattered radiation dose (660) in dynamic imaging by the radiographic imaging apparatus of the mobile type based on the setting.

3. The radiographic imaging apparatus of the mobile type according to claim 1, wherein
the scattered radiation dose map illustrates a region (510, 520) with a radiation dose equal to or less than a predetermined radiation dose or a region (420, 430, 440) with a radiation dose exceeding the predetermined radiation dose.

4. The radiographic imaging apparatus of the mobile type according to claim 3, wherein
the predetermined radiation dose is changeable.

5. The radiographic imaging apparatus of the mobile type according to claim 1, wherein
the scattered radiation dose map is a scattered radiation dose map in which the scattered radiation dose is overlapped on a base map corresponding to the setting.

6. The radiographic imaging apparatus of the mobile type according to claim 1, wherein
the setting(s) includes at least one of an imaging location, an imaging condition (620) for a radiation generating apparatus, and/or an imaging body position.

7. The radiographic imaging apparatus of the mobile type according to claim 6, wherein
the setting(s) includes an arrangement of a shielding material at the imaging location.

8. A program that causes a computer to execute:
inputting a setting of moving image capturing;
estimating a scattered radiation dose (660) of a radiographic imaging apparatus of a mobile type based on the setting; and
outputting a scattered radiation dose map based on the scattered radiation dose (660).

9. The program according to claim 8, wherein
in the estimating, the scattered radiation dose (660) in dynamic imaging by the radiographic imaging apparatus of the mobile type is estimated based on the setting.

10. The program according to claim 8, wherein
the scattered radiation dose map illustrates a region (510, 520) with a radiation dose equal to or less than a predetermined radiation dose or a region (420, 430, 440) with a radiation dose exceeding the predetermined radiation dose.

11. The program according to claim 10, wherein
the predetermined radiation dose is changeable.

12. The program according to claim 8, wherein
the scattered radiation dose map is a scatted radiation dose map in which the scattered radiation dose is overlapped on a base map corresponding to the setting.

13. The program according to claim 8, wherein
the setting(s) includes at least one of an imaging location, an imaging condition (620) for a radiation generating apparatus, and/or an imaging body position.

14. The program according to claim 13, wherein
the setting(s) includes an arrangement of a shielding material at the imaging location.

15. A moving image capturing method, comprising:
inputting a setting of moving image capturing;
estimating a scattered radiation dose (660) of a radiographic imaging apparatus of a mobile type based on the setting;
outputting a scattered radiation dose map based on the scattered radiation dose (660); and
capturing a moving image.
